# EUROPÄISCHE PATENTSCHRIFT

(11) **EP 2 560 602 B1**
(45) Veröffentlichungstag und Bekanntmachung des Hinweises auf die Patenterteilung: **11.06.2014**
(21) Anmeldenummer: 11713839.6
(22) Anmeldetag: 12.04.2011
(51) Int. Cl.: A61J 3/07, B65B 43/52

(54) **VORRICHTUNG ZUM FÜLLEN UND VERSCHLIEßEN VON MIT WENIGSTENS EINEM FÜLLGUT BEFÜLLTEN KAPSELN**
DEVICE FOR FILLING AND CLOSING CAPSULES FILLED WITH AT LEAST ONE FILLER
DISPOSITIF POUR LE REMPLISSAGE ET LA FERMETURE DE CAPSULES REMPLIES D'AU MOINS UNE MATIÈRE DE REMPLISSAGE

(30) Priorität: 22.04.2010 DE 102010028125
(43) Veröffentlichungstag der Anmeldung: 27.02.2013
(73) Patentinhaber: Robert Bosch GmbH, 70442 Stuttgart (DE)
(72) Erfinder: SCHMIED, Ralf, 71691 Freiberg (DE); COMANNS, Udo, 73630 Remshalden (DE); FRANCK, Thomas, 73547 Lorch-Weitmars (DE); EISTERT, Olaf, 73102 Birenbach (DE); BOEHRINGER, Walter, 73630 Remshalden (DE); REUM, Klaus, 73630 Remshalten (DE); MIHALEK, Thomas, 73660 Urbach (DE)
(86) Internationale Anmeldenummer: PCT/EP2011/055734
(87) Internationale Veröffentlichungsnummer: WO 2011/131521

(56) Entgegenhaltungen:
- EP-A1- 2 135 595
- DE-U1-202008 007 921
- US-A- 2 764 863

## Beschreibung

### Stand der Technik

Die Erfindung betrifft eine Vorrichtung zum Füllen und Verschließen von mit wenigstens einem Füllgut befüllten Kapseln nach dem Oberbegriff des Anspruchs 1.

Eine derartige Vorrichtung ist aus der DE 10 2008 013 403 A1 der Anmelderin bekannt und dient zum Füllen und Verschließen von insbesondere Hartgelatinekapseln mit einem Füllgut, insbesondere mit einem pulverförmigen Arzneimittel. Hierbei sind die einzelnen Stationen der Vorrichtung an einem schrittweise gedrehten Förderrad angeordnet, wobei die Hartgelatinekapseln jeweils in den Bereich der einzelnen Stationen gefördert werden, um an diesen entsprechende Behandlungsprozesse vornehmen zu können. Eine derartige, bekannte Vorrichtung ist hinsichtlich ihrer Baugröße und Leistung insofern beschränkt, als dass bei zunehmendem Durchmesser und größerer Drehzahl des Förderrades an den sich mitdrehenden Aufnahmeeinheiten für die Kapseln unerwünscht hohe Kräfte bzw. Beschleunigungen auftreten. Weiterhin ist die Zugänglichkeit im zentralen Bereich des Förderrades bei größer werdendem Durchmesser des Förderrades beschränkt.

Aus der DE 10 2005 059 371 A1 sind ferner Kapseln zum Einsatz insbesondere in der Landwirtschaft bekannt, welche mit einem Saatgut sowie anderen Füllgütern befüllt sind, um ein optimales Wachstum oder sonstige Eigenschaften des Saatgutes zu bewirken. Derartige Kapseln können je nach Verwendung bzw. Füllgut und Füllgutmenge eine größere Größe bzw. ein größeres Volumen aufweisen als die aus dem Arzneimittelbereich bekannten Kapseln. Auch die Handhabung derartiger, größerer Kapseln ist auf der bekannten Vorrichtung erschwert, da dann die einzelnen Stationen jeweils größer bauen müssten, was den Raumbedarf zusätzlich vergrößert und somit die Zugänglichkeit der Vorrichtung weiter erschwert.

Aus der US 2,764,863 A ist bereits eine gattungsgemäße Kapselfüllmaschine bekannt. Diese bevorzugt kontinuierlich betreibbare Kapselfüllmaschine kommt für eine Vielzahl von Kapseln zum Einsatz. Die Kapselfüllmaschine führt die gewünschten Operationen wie trennen, füllen, verbinden und auswerfen aus.

### Offenbarung der Erfindung

Ausgehend von dem dargestellten Stand der Technik liegt der Erfindung die Aufgabe zugrunde, eine Vorrichtung zum Füllen und Verschließen von mit wenigstens einem Füllgut befüllten Kapseln nach dem Oberbegriff des Anspruchs 1 derart weiterzubilden, dass diese insbesondere eine möglichst große Leistung sowie eine gute Zugänglichkeit zu deren einzelnen Stationen aufweist. Diese Aufgabe wird bei einer Vorrichtung mit den Merkmalen des Anspruchs 1 gelöst. Der Erfindung liegt dabei die Idee zugrunde, durch die Ausbildung der Fördereinrichtung mit mindestens einem endlos ausgebildeten Fördertrum die Fördereinrichtung als geradlinig ausgebildete Fördereinrichtung auszubilden, an deren Förderstrecke die einzelnen Stationen zur Behandlung der Kapseln angeordnet sind. Hierbei dienen insbesondere rechteckförmige bzw. leistenartige Aufnahmeplatten der Befestigung bzw. dem einfachen Austausch von Aufnahmeeinheiten zur Formatanpassung an unterschiedliche Größen der Kapseln. Durch die Ausbildung der Fördereinrichtung als lineare Fördereinrichtung lässt sich die Leistungsfähigkeit der Vorrichtung prinzipiell einfach anpassen bzw. steigern, indem die Länge der Vorrichtung vergrößert wird, um z.B. zusätzliche Füllstationen entlang des Förderwegs der Kapseln anzuordnen. Weiterhin ist durch die lineare Bauweise der Fördereinrichtung die Bautiefe der Vorrichtung relativ gering, so dass eine einfache Erreichbarkeit zu den einzelnen Stationen gegeben ist, die ggf. von der Rück- sowie der Vorderseite der Vorrichtung ermöglicht wird.

Vorteilhafte Weiterbildungen der erfindungsgemäßen Vorrichtung zum Füllen und Verschließen von mit wenigstens einem Füllgut befüllten Kapseln sind in den Unteransprüchen angegeben.

Eine einfache Möglichkeit zur Leistungssteigerung der Vorrichtung ist gegeben, wenn die Fördereinrichtung wenigstens zwei Fördertrume aufweist, wenn die

Aufnahmeplatten die wenigstens zwei Fördertrume überspannen und, wenn je Aufnahmeplatte insbesondere mehrere, quer zur Förderrichtung zueinander fluchtend angeordnete Aufnahmeeinheiten an den Aufnahmeplatten vorgesehen sind. Damit wird bei gegebener Länge der Vorrichtung die Möglichkeit geschaffen, pro Fördertakt jeweils mehrere Aufnahmeeinheiten gleichzeitig behandeln zu können, wodurch sich die Leistung der Vorrichtung erhöht. Gleichzeitig ist die Fertigung der Aufnahmeeinheiten bei einer bestimmten Leistung der Vorrichtung vereinfacht, da die Aufnahmeeinheiten kleiner bauen können und auf den Aufnahmeplatten einfach zueinander ausgerichtet werden können, so dass die Fertigungsgenauigkeit der einzelnen Aufnahmeeinheiten reduziert werden kann. Dies betrifft insbesondere die Anordnung bzw. Ausrichtung der Aufnahmebohrungen für die Kapseln in den Aufnahmeeinheiten.

In einer bevorzugten Ausführungsform der Erfindung ist es vorgesehen, dass in jeder der Aufnahmeeinheiten mehrere, in Förderrichtung nacheinander angeordnete Reihen mit Aufnahmebohrungen für die Kapseln ausgebildet sind und, dass jeder Reihe von Aufnahmebohrungen eine separate Sortiereinrichtung zum Zuführen der leeren Kapseln zugeordnet ist, wobei die separaten Sortiereinrichtungen in Förderrichtung jeweils einen Abstand zueinander aufweisen, der der Länge eines Fördertaktes der Fördereinrichtung, verringert oder verlängert um den Abstand zweier Reihen an Aufnahmebohrungen zueinander entspricht. Das bedeutet, dass die Aufnahmebohrungen der Aufnahmeeinheiten in mehreren Schritten bzw. während mehrerer Takte mit den Kapseln befüllt werden, was die Möglichkeit schafft, relativ einfach ausgebildete Zuführeinrichtungen für die Kapseln vorzusehen.

Während das Zuführen der leeren Kapseln zu den Aufnahmeeinheiten in einem Zustand der Aufnahmeeinheiten erfolgt, in der das jeweils untere und obere Aufnahmesegment der Aufnahmeeinheit übereinander fluchtend angeordnet sind, ist es für die weiteren Behandlungen, insbesondere das Befüllen der Kapselunterteile erforderlich, das obere Aufnahmesegment von dem unteren Aufnahmesegment zu trennen, um damit gleichzeitig die Kapseln zu öffnen. Ebenso müssen die Aufnahmesegmente nach dem Befüllen der Kapselunterteile wieder zusammengeführt werden. Hierbei ist es in einer konstruktiven Ausgestaltung der Erfindung vorgesehen, dass beiden Umsetzeinrichtungen zum Trennen bzw. wieder Zusammenführen der Aufnahmesegmente für jede Aufnahmeeinheit zwei, an gegenüberliegenden Seiten der Aufnahmeeinheit angeordnete Transportsterne aufweist, die in einer horizontalen Achse drehbar gelagert sind, wobei die Transportsterne jeweils wenigstens einen Förderarm aufweisen, der mit einem oberen Aufnahmesegment zusammenwirkt und dieses aufnimmt, wobei die Länge des Förderarms der Länge eines Fördertakts der ersten Fördereinrichtung verringert oder verlängert um wenigstens die Breite der unteren Aufnahmesegmente in Förderrichtung entspricht. Derartige Umsetzeinrichtungen ermöglichen beim taktweisen Betrieb der Fördereinrichtung eine zusätzliche bzw. separate Bewegung für das obere Aufnahmesegment mit relativ geringem Aufwand bzw. ohne dass hierzu der Bewegungsablauf der Fördereinrichtung beeinflusst wird.

Während des Produktionsprozesses bzw. des Füllvorganges der Kapseln kann es vorkommen, dass einzelne Kapseln beispielsweise beschädigt oder nicht korrekt befüllt wurden. Daher ist es erforderlich, diese Schlecht-Kapseln auszuscheiden. Hierbei ist es in einer weiteren Ausgestaltung der Erfindung vorgesehen, dass der Ausschleusseinrichtung für die befüllten Gut-Kapseln eine Ausscheideeinrichtung für Schlecht-Kapseln vorgeordnet ist und, dass die Ausscheideeinrichtung Ausschiebestempel aufweist, die zumindest die Schlecht-Kapseln aus den Aufnahmebohrungen der Aufnahmeeinheiten auf eine weitere Fördereinrichtung zumindest mittelbar ausschiebt, die die Schlecht-Kapseln aus dem Bereich der ersten Fördereinrichtung fördert. Eine derartige Ausbildung stellt sicher, dass Schlecht-Kapseln zuverlässig ausgeschieden werden.

In einer konstruktiv besonders bevorzugten Ausführungsform ist es dabei vorgesehen, dass die Ausscheideeinrichtung ein Ausschiebeprisma aufweist, das in einer Stillstandsphase der ersten Fördereinrichtung in Überdeckung mit einer Aufnahmeeinheit und oberhalb der Aufnahmeeinheit angeordnet ist, dass das Ausschiebeprisma Durchgangsbohrungen aufweist, die in Deckung mit den Aufnahmebohrungen der Aufnahmeeinheit angeordnet sind und, dass das Prisma wenigstens eine insbesondere schräg zur Horizontalen angeordnete Rutschfläche aufweist, von der Schlecht-Kapseln auf die weitere Fördereinrichtung gelangen. Durch das Prisma mit seiner schräg angeordneten Rutschfläche wird somit ein einfaches Überleiten der ausgeschiedenen Kapseln auf die weitere Fördereinrichtung ermöglicht, wobei das Überführen durch Herabrutschen der Kapseln an der Rutschfläche erfolgt.

Weitere Vorteile, Merkmale und Einzelheiten der Erfindung ergeben sich aus der nachfolgenden Beschreibung bevorzugter Ausführungsbeispiele sowie anhand der Zeichnungen.

Diese zeigen in:
- Fig. 1: eine vereinfachte perspektivische Seitenansicht auf eine erfindungsgemäße Vorrichtung zum Füllen und Verschließen von Kapseln,
- Fig. 2: einen vereinfachten Schnitt im Bereich von Sortierstationen,
- Fig. 3: eine Draufsicht auf untere Aufnahmesegmente im Bereich der Sortierstationen,
- Fig. 4 bis Fig. 9: jeweils perspektivische Ansichten auf eine vereinfacht dargestellte Umsetzstation zum Trennen von oberen Aufnahmesegmenten von unteren Aufnahmesegmenten,
- Fig. 10: einen Schnitt durch eine Pelettfülleinrichtung,
- Fig. 11 und Fig. 12: vereinfachte Darstellungen einer modifizierten Fülleinrichtung unter Verwendung von pneumatischen Saugmitteln,
- Fig. 13: Schnitte im Bereich einer Kontrollstation zur Prüfung der korrekten Anwesenheit von Füllgütern,
- Fig. 14: einen Schnitt durch die Vorrichtung gemäß Fig. 1 im Bereich einer Schließstation und
- Fig. 15 und Fig. 16: perspektivische, teilweise geschnittene Ansichten einer Ausscheidestation für Schlecht-Kapseln.

Gleiche Bauteile bzw. Bauteile mit gleicher Funktion sind in den Figuren mit identischen Bezugsziffern versehen.

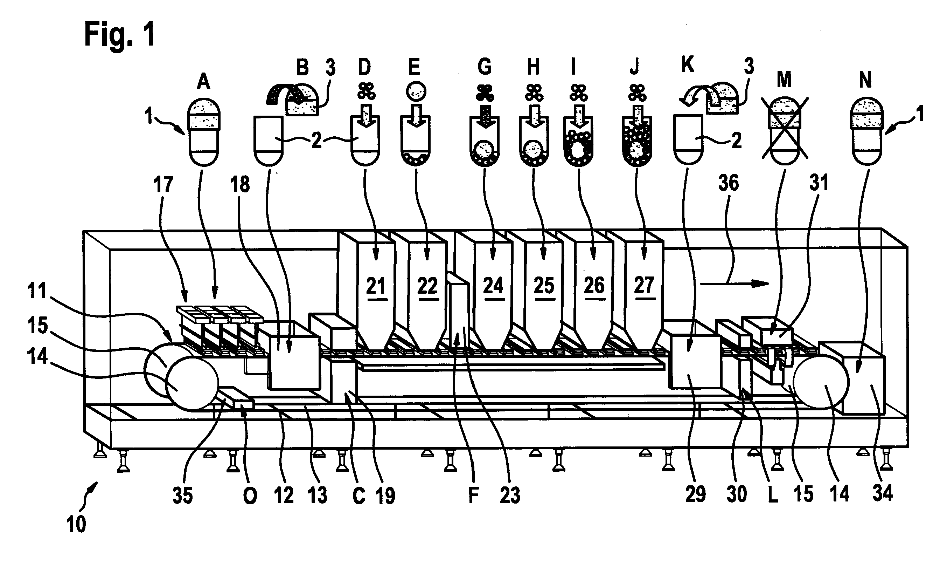

In der Fig. 1 ist eine erfindungsgemäße Vorrichtung 10 zum Füllen und Verschließen von Kapseln 1 dargestellt. Die Kapseln 1 bestehen jeweils aus einem Kapselunterteil 2 und einem Kapseloberteil 3, wobei das Kapseloberteil 3 in geschlossenem Zustand der Kapsel 1 klemmend auf dem Kapselunterteil 2 angeordnet ist. Derartige Kapseln 1 sind beispielsweise aus dem Pharmaziebereich oder sonstigen Bereichen bekannt, wobei das Material der Kapseln 1 derart ausgebildet ist, dass sich dieses beispielsweise unter dem Einfluss von Medien, wie Flüssigkeiten oder Luft, mit der Zeit zersetzt, um das in den Kapseln 1 gespeicherte Füllgut an die Umgebung abzugeben. Im konkreten Anwendungsfall dient die Vorrichtung 10 zum Füllen von Kapseln 1 mit mehreren, insbesondere unterschiedlichen Füllgütern aus dem landwirtschaftlichen Bereich, wobei die Füllgüter insbesondere ein Saatgut sowie weitere Zusatzstoffe, wie beispielsweise Dünger, Pestizide oder ähnliches, umfassen.

Die Vorrichtung 10 weist eine Fördereinrichtung 11 auf, die als endlos ausgebildeter Bauweise ausgebildet ist. Hierzu weist die Fördereinrichtung 11 beispielhaft zwei, parallel zueinander angeordnete Fördertrume 12, 13 in Form von beispielsweise Fördergurten oder Förderketten auf. Die Fördertrume 12, 13 sind um vorzugsweise jeweils miteinander gekoppelte Umlenkräder 14, 15 geführt, von denen wenigstens eins der Umlenkräder 14, 15 taktweise angetrieben wird.

Ergänzend wird erwähnt, dass die Fördereinrichtung 11 weitere, in der Fig. 1 nicht näher erkennbare Stützmittel wie Stützräder, Führungsschienen oder ähnliches zur Führung der beiden Fördertrume 12, 13 aufweisen kann. Mittels der Fördereinrichtung 11 werden die Kapseln 1 taktweise einer Reihe von Stationen A bis O zugeführt, an denen die Kapseln 1 unterschiedlichen Behandlungen bzw. Prozessschritten unterworfen werden. Hierbei ist die Station A als Sortiereinrichtung 17 ausgebildet, die Station B als erste Umsetzeinrichtung 18 und die Station C als Fehlkapselausscheideeinrichtung 19. An die Fehlkapselausscheideeinrichtung 19 schließen sich die beiden Stationen D und E an, die jeweils als Fülleinrichtung 21 und 22 ausgebildet sind. Auf die Fülleinrichtung 22 folgt an der Station F eine Prüfeinrichtung 23, gefolgt von weiteren vier Stationen G bis J, jeweils ausgebildet als Fülleinrichtungen 24 bis 27. Nach der letzten Fülleinrichtung 27 folgt eine weitere, zweite Umsetzeinrichtung 29 an der Station K, gefolgt von einer Schließeinrichtung 30 an der Station L, einer Ausscheideeinrichtung 31 für Schlecht-Kapseln 1 an der Station M und einer im Umlenkbereich der Fördereinrichtung 11 angeordneten Ausschleusseinrichtung 34 für Gut-Kapseln 1 an der Station N. Zuletzt ist im Bereich des Rücklaufes der Fördertrume 12, 13 eine Reinigungseinrichtung 35 an der Station O angeordnet.

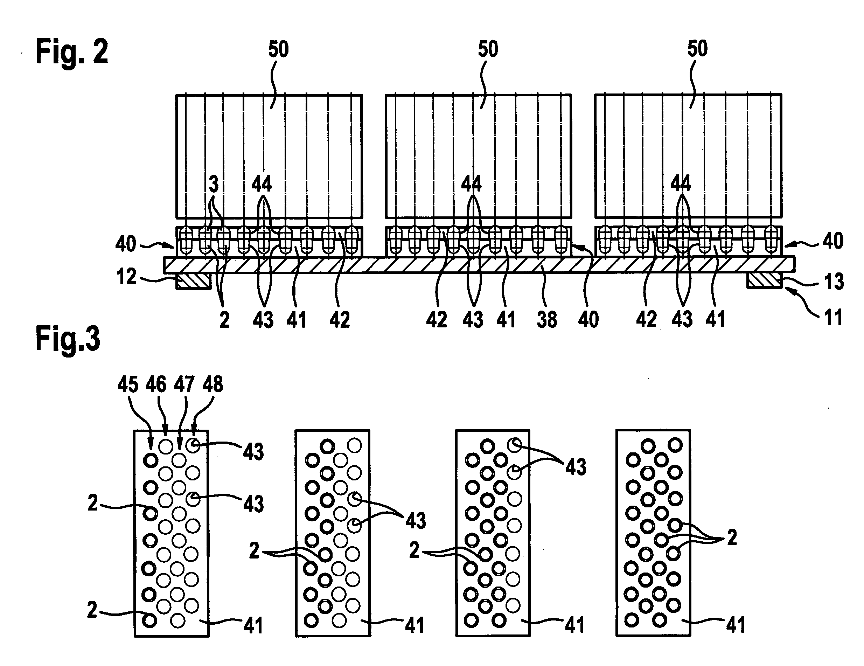

Wie man am besten anhand einer Zusammenschau der Fig. 2 und 3 erkennt, sind die beiden Fördertrume 12, 13 der Fördereinrichtung 11 quer zur Förderrichtung 36 der oberen Trume 12, 13 der Fördereinrichtung 11 von insbesondere rechteckigen bzw. leistenartigen Aufnahmeplatten 38 überspannt, die in gleichmäßigen Abständen zueinander angeordnet sind, wobei die Abstände der Aufnahmeplatten 38 in Förderrichtung 36 der Fördereinrichtung 11 betrachtet insbesondere der Strecke eines Fördertaktes der Fördereinrichtung 11 entsprechen. Die Aufnahmeplatten 38 dienen der auswechselbaren bzw. der austauschbaren, da formatabhängigen Befestigung von Aufnahmeeinheiten 40. Wie am besten anhand der Fig. 2 erkennbar ist, sind im Ausführungsbeispiel quer zur Förderrichtung 36 der Fördereinrichtung 11 betrachtet drei Aufnahmeeinheiten 40 nebeneinander angeordnet. Jede der Aufnahmeeinheiten 40 dient der Aufnahme einer Vielzahl von Kapseln 1 und besteht aus einem unteren Aufnahmesegment 41 und einem oberen Aufnahmesegment 42. Im unteren Aufnahmesegment 41 sind untere Aufnahmebohrungen 43 und im oberen Aufnahmesegment 42 obere Aufnahmebohrungen 44 ausgebildet. Die unteren Aufnahmebohrungen 43 wirken mit den Kapselunterteilen 2 und die oberen Aufnahmebohrungen 44 mit den Kapseloberteilen 3 zusammen.

Wie man insbesondere anhand der Fig. 3 am Beispiel der unteren Aufnahmesegmente 41 erkennt, sind die unteren Aufnahmebohrungen 43 (ebenso wie die oberen Aufnahmebohrungen 44) in einer Richtung quer zur Förderrichtung 36 der Fördereinrichtung 11 in gleichmäßigen Abständen mittig versetzt zueinander angeordnet. Im Ausführungsbeispiel sind jeweils sieben untere bzw. obere Aufnahmebohrungen 43, 44 quer zur Förderrichtung 36 in den Aufnahmesegmenten 41, 42 vorgesehen. Die sieben Aufnahmebohrungen 43, 44 sind jeweils in Form von Reihen 45 bis 48 in den Aufnahmesegmenten 41, 42 angeordnet. Die Aufnahmebohrungen 43, 44 der Reihen 45 und 47 sowie die Reihen 46 und 48 sind quer zur Förderrichtung 36 jeweils fluchtend zueinander angeordnet.

Die Sortiereinrichtung 17 umfasst vier, in Fördereinrichtung 36 hintereinander angeordnete Sortierer 50 für jede der Aufnahmeeinheiten 40. Die Sortierer 50 sind von an sich bekannter Bauart und dienen dazu, jeweils eine Kapsel 1, bestehend aus Kapselunterteil 2 und Kapseloberteil 3 in die beiden übereinander angeordneten Aufnahmebohrungen 43, 44 der Aufnahmesegmente 41, 42 der Aufnahmeeinheiten 40 überzuschieben bzw. einzuschieben. Wesentlich hierbei ist, dass jeder der Sortierer 50 jeweils zum gleichzeitigen Einschieben von Kapseln 1 in einer der Reihen 45 bis 48 dient. Im dargestellten Ausführungsbeispiel dient ein Sortierer 50 zum Einschieben der Kapseln 1 in die Reihe 45, ein Sortierer 50 für die Reihe 46, ein Sortierer 50 für die Reihe 47 und ein Sortierer 50 für die Reihe 48. Hierbei entspricht der Abstand der Sortierer 50 in Förderrichtung 36 der Fördereinrichtung 11 betrachtet jeweils der Länge einer Fördertaktes der Fördereinrichtung 11, vergrößert um den Abstand von aufeinanderfolgenden Reihen 45 bis 48 in Förderrichtung 36. Dadurch werden die Reihen 45 bis 48 während vier aufeinanderfolgender Fördertakte der Fördereinrichtung 11 mit Kapseln 1 befüllt, wie dies anhand der Fig. 3 erkennbar ist.

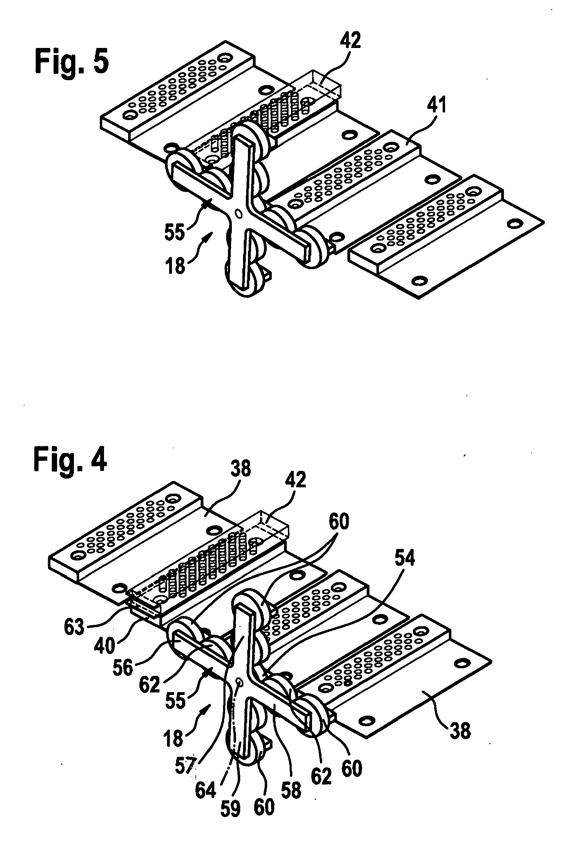

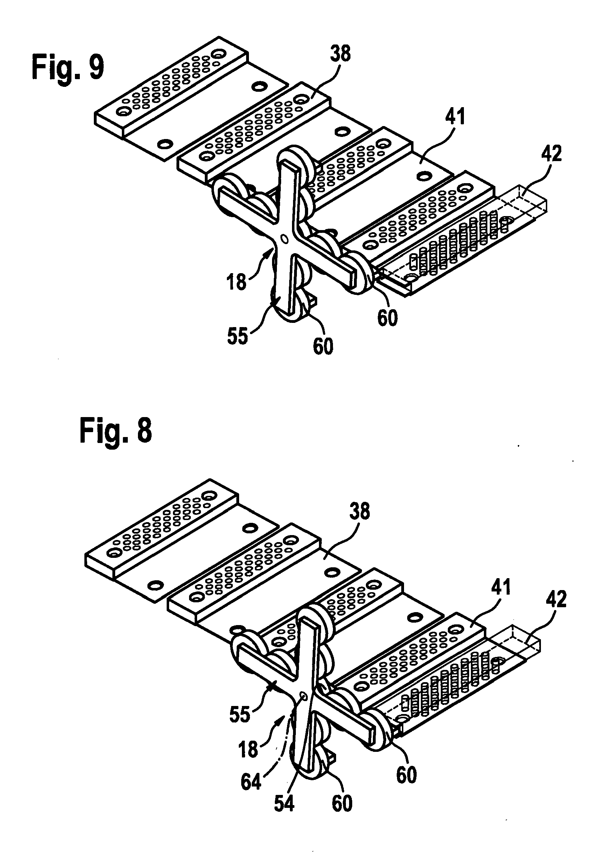

Nachdem die Kapseln 1 in die Aufnahmeeinheiten 40 mittels der Sortiereinrichtung 17 eingeschoben wurden, erfolgt mittels Unterdruck die Trennung der Kapselunterteile 2 von den Kapseloberteilen 3. Hierbei verbleiben die Kapseloberteile 3 im oberen Aufnahmesegment 42 und die Kapselunterteile 2 werden in das unteren Aufnahmesegment 41 gezogen. Anschließend müssen die oberen Aufnahmesegmente 42 von den unteren Aufnahmesegmenten 41 getrennt werden, um im weiteren Verlauf der Förderung der Aufnahmeeinheiten 40 die Kapselunterteile 2 mit den entsprechenden Füllgütern befüllen zu können. Hierzu ist die in den Fig. 4 bis 9 näher dargestellte erste Umsetzeinrichtung 18 vorgesehen. Die Umsetzeinrichtung 18 weist für jede der Aufnahmeeinheiten 40 an den beiden gegenüberliegenden Stirnseiten der Aufnahmeeinheiten 40 jeweils einen Transportstern 55 auf, von denen in den Fig. 4 bis 9 der Einfachheit halber lediglich ein Transportstern 55 dargestellt ist. Der Transportstern 55 weist im Ausführungsbeispiel vier, um jeweils 90° zueinander versetzt angeordnete Förderarme 56 bis 59 auf. Die Transportarme 56 bis 59 weisen auf der den Aufnahmeeinheiten 40 zugewandten Seite jeweils zwei in den Transportarmen 56 bis 59 drehbar gelagerte Aufnahmerollen 60 auf, an denen auf der der Aufnahmeeinheit 40 zugewandten Seite jeweils eine in den Figuren nicht erkennbare Aufnahme ausgebildet ist, die mit einer Aufnahme 63 an der jeweiligen Stirnfläche des oberen Aufnahmesegments 42 formschlüssig zusammenwirkt. Hierzu ist die Drehachse 64 der Transportsterne 55 in einer Höhe angeordnet, die mit der Höhe der oberen Aufnahmesegmente 42 bzw. den Aufnahmen 63 der oberen Aufnahmesegmente 42 ausgerichtet ist.

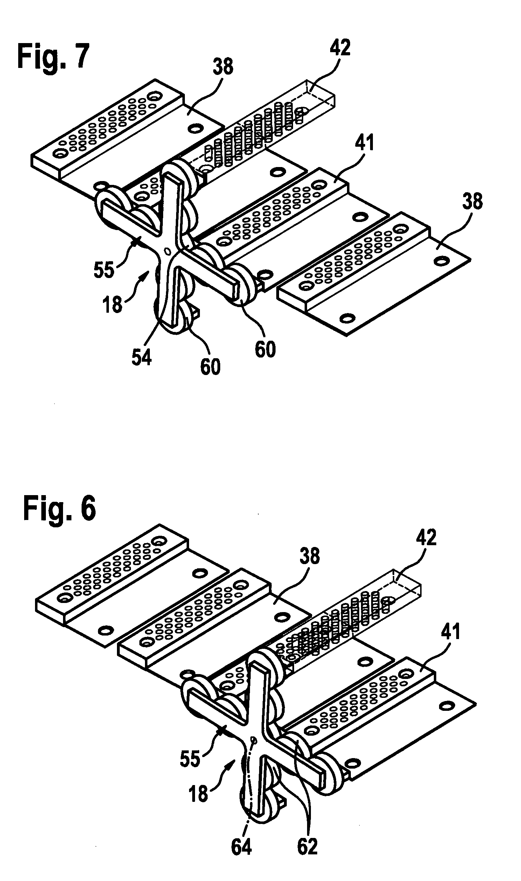

Anhand der Abfolge der Fig. 4 bis 9 ist erkennbar, wie eine Aufnahmeeinheit 40 bei einem Fördertakt der Fördereinrichtung 11 mit ihrem oberen Aufnahmesegment 42 in Wirkverbindung mit der Aufnahme 63 des Förderarms 56 gerät (Fig. 5). Beim nachfolgenden Fördertakt entsprechend den Fig. 6 bis 9 wird das obere Aufnahmesegment 42 von dem unteren Aufnahmesegment 41 mittels der Transportarme 55 abgehoben und dabei in Förderrichtung 36 der Fördereinrichtung 11 betrachtet vor das untere Aufnahmesegment 42 auf die Aufnahmeplatte 38 abgesetzt. Hierzu ist es wesentlich, dass der Abstand der Aufnahme 63 von der Drehachse 64 des Transportarms 55 um die Breite zumindest des unteren Aufnahmesegmentes 41 verlängert ausgebildet ist. Weiterhin ist erkennbar, dass das obere Aufnahmesegment 42 durch die Aufnahmerollen 60, die über Zwischenräder 62 von einem zentralen Sonnenrad 54 bewegt werden, während ihres Transports im Transportstern 55 jeweils horizontal ausgerichtet bleiben.

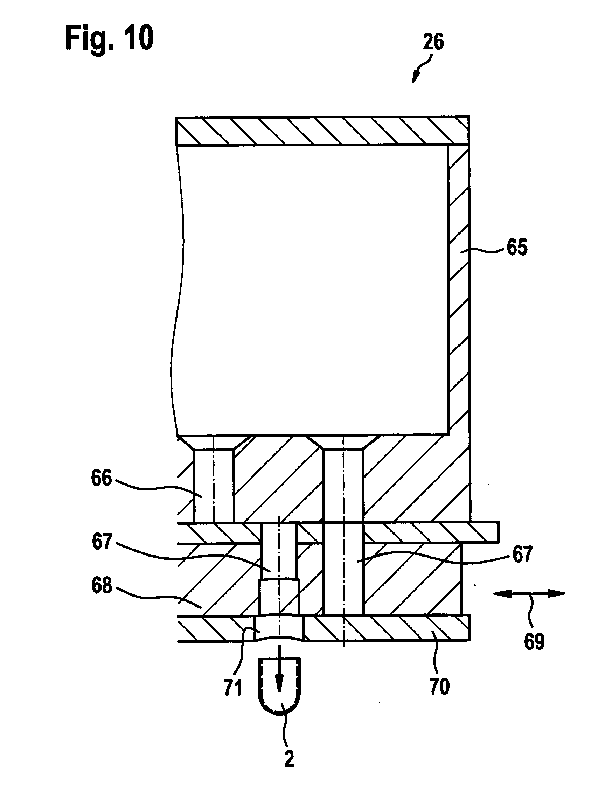

In der Fig. 10 ist beispielhaft eine der Fülleinrichtungen 21, 22 bzw. 24 bis 27 dargestellt. Die anhand der Fülleinrichtung 26 beschriebene Fülleinrichtung weist einen Produktspeicherbehälter 65 auf, in dem zu dosierendes Produkt in Form von Pellets (nicht dargestellt) gespeichert ist. Am Grund des Produktspeicherbehälters 65 sind Durchgangsbohrungen 66 ausgebildet, die mit Durchgangsbohrungen 67 eines Dosierschiebers 68 zusammenwirken. Hierbei wird über den Durchmesser sowie die Höhe bzw. Dicke der Durchgangsbohrungen 67 in dem Dosierschieber 68 die in ein Kapselunterteil 2 einzudosierende Menge an Füllgut bestimmt. Der in einer Richtung parallel zur Förderrichtung 36 der Fördereinrichtung 11 in Richtung des Doppelpfeils 69 hin- und herbewegbare Dosierschieber 68 ist in seiner einen Endstellung mit den unteren Aufnahmebohrungen 43 des unteren Aufnahmesegments 41 und in seiner anderen Endstellung mit den Durchgangsbohrungen 66 im Produktspeicherbehälter 65 ausgerichtet. Weiterhin ist unterhalb des Dosierschiebers 68 noch eine ortsfeste Dosierplatte 70 mit Durchgangsbohrungen 71 angeordnet, wobei die Durchgangsbohrungen 71 in der Stillstandsphase der Fördereinrichtung 11 mit den unteren Aufnahmebohrungen 43 bzw. den Kapselunterteilen 2 ausgerichtet sind. Die Arbeitsweise der Fülleinrichtung 26 ist wie folgt: Wenn die Durchgangsbohrungen 66 des Produktspeicherbehälters 65 mit den Durchgangsbohrungen 67 des Dosierschiebers 68 ausgerichtet ist, fällt Füllgut aus dem Produktspeicherbehälter 65 in die Durchgangsbohrungen 67 des Dosierschiebers 68. Hierbei bildet die Dosierplatte 70 eine untere Begrenzung für die Dosierbohrungen 67 aus. Bei der nachfolgenden Bewegung des Dosierschiebers 68 in Förderrichtung 36 werden die Durchgangsbohrungen 67 von den Durchgangsbohrungen 66 getrennt und durch den Dosierschieber 68 verschlossen, so dass das Produktvolumen vom Dosierschieber 68 in Richtung der Durchgangsbohrungen 71 der Dosierplatte 70 gefördert wird. Sobald die Durchgangsbohrungen 67 des Dosierschiebers 68 mit den Durchgangsbohrungen 71 der Dosierplatte 70 fluchten, rutscht das Füllgut infolge der Schwerkraft durch die Durchgangsbohrungen 71 der Dosierplatte 70 in die unterhalb der Dosierplatte 70 angeordneten Kapselunterteile 2. Wesentlich ist noch, dass die Durchgangsbohrungen 67 im Dosierschieber 68 derart angeordnet bzw. ausgerichtet sind, dass bei einer Übernahme von Füllgut aus dem Produktspeicherbehälter 65 die entsprechende Durchgangsbohrung 67 sich im Bereich zweier Aufnahmebohrungen 43 zwischen den Reihe 45 bis 48 befindet, so dass ein genügend großer horizontaler Verfahrweg für den Dosierschieber 68 ermöglicht wird, bis dieser sein Füllgut in das entsprechende Kapselunterteil 2 abgibt.

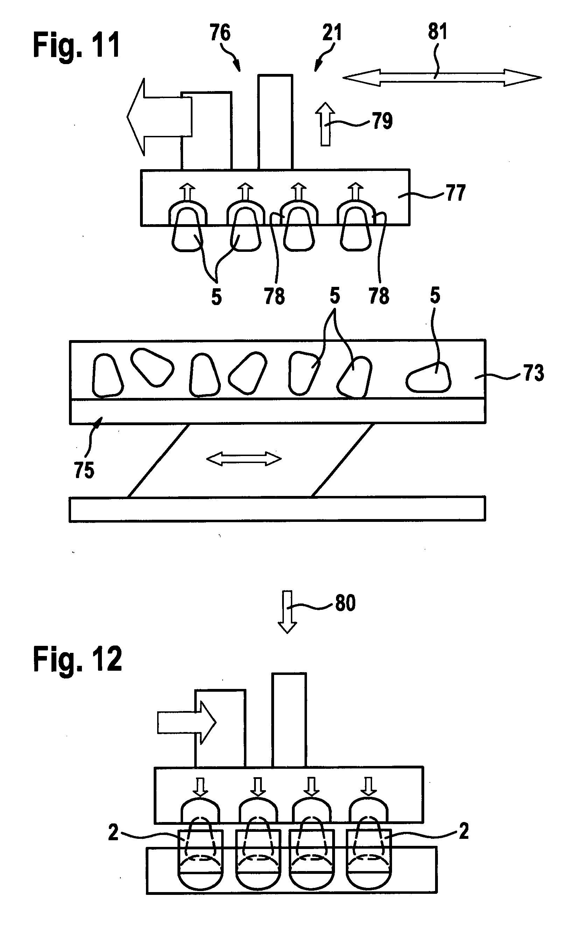

In den Fig. 11 und 12 wird anhand der Fülleinrichtung 21 beispielhaft das Abgeben von relativ großkörnigem Produkt 5 in die Kapselunterteile 2 beschrieben. Hierbei ist das Produkt 5 in einem schalenförmigen Aufnahmebehälter 73 eines Vibrationsfördertopfes 75 angeordnet. Ferner ist eine Saugeinrichtung 76 mit einem Saugkopf 77 vorgesehen, in dem für jedes Produkt 5 eine Aufnahme 78 ausgebildet ist. Jeder der Aufnahmen 78 ist über eine nicht dargestellte Verbindung mit einer Unterdruckquelle verbunden. Der Saugkopf 77 ist wiederum entsprechend der Pfeile 79 und 80 auf- bzw. abbewegbar sowie entsprechend des Doppelpfeils 81 horizontal bewegbar. Zum Überführen von Produkt 5 aus dem Aufnahmebehälter 73 in die Kapselunterteile 2 taucht der Saugkopf 77 bei eingeschalteter Unterdruckquelle in den Aufnahmebehälter 73 ein, übernimmt in jeder Aufnahme 78 ein Produkt 5 und übergibt anschließend durch in Deckung bringen und Absenken das Produkt 5 in die Kapselunterteile 2, wobei dann die Unterdruckquelle abgeschaltet wird.

Ergänzend wird erwähnt, dass die anhand der Fülleinrichtungen 21 und 26 dargestellten Wirkprinzipien lediglich exemplarisch sind und durch gängige, dem jeweiligen Füllgut bzw. Produkt angepasste Methoden ersetzt werden können.

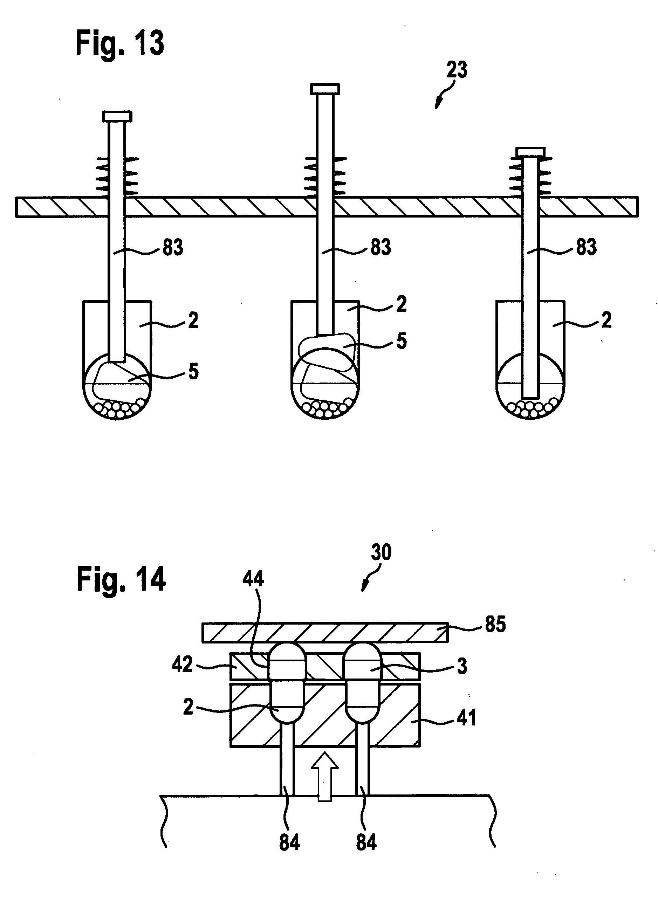

In der Fig. 13 ist das Wirkprinzip der Prüfeinrichtung 23 dargestellt. Hierbei werden die Kapselunterteile 2 jeweils auf die Anwesenheit zumindest eines Produkts 5 überprüft. Die Prüfeinrichtung 23 weist hierzu einen federbelasteten, auf- und abbewegbaren Prüfstempel 83 auf, der beim Vorhandensein eines Produkts 5 bis in eine bestimmte Position angehoben wird, die mittels nicht dargestellter Detektionsmittel erkennbar ist (linkes Bildteil der Fig. 13). Sollte entsprechend dem mittleren Bildteil der Fig. 13 hingegen zu viel Produkt 5 in dem Kapselunterteil 2 vorhanden sein, so wird der Prüfstempel 83 um einen Betrag angehoben, der größer ist als bei einer korrekten Menge an Produkt 5. Daraus wird auf das Vorhandensein von zuviel Produkt 5 geschlossen. Ist hingegen entsprechend dem rechten Bildteil der Fig. 13 kein Produkt 5 im Kapselunterteil 2 vorhanden, so wird der Prüfstempel 83 überhaupt nicht angehoben, was ebenfalls detektiert wird.

Sobald erkannt wurde, dass entweder zu viel Produkt 5 oder aber kein Produkt 5 in dem jeweiligen Kapselunterteil 2 eindosiert wurde, wird dies der Steuereinrichtung der Vorrichtung 10 mitgeteilt, welche im weiteren Verlauf vorzugsweise eine komplette Reihe 45 bis 48 mit Kapselunterteilen 2 bzw. Kapseln 1 ausschleust. Selbstverständlich liegt es auch im Rahmen der Erfindung, die genaue Position der jeweils betroffenen Kapsel 1 bzw. des jeweils betroffenen Kapselunterteils 2 zu detektieren und im weiteren Verlauf gezielt jeweils nur die betroffene Kapsel 1 bzw. das betroffene Kapselunterteil 2 auszuschleusen.

Die zweite, im Einzelnen nicht näher dargestellte Umsetzeinrichtung 29 an der Station K ist prinzipiell entsprechend der Umsetzeinrichtung 18 an der Station B ausgebildet. Wesentlich hierbei ist nur, dass der entsprechende Transportstern 55 bzw. dessen Aufnahme 63 in einem Abstand von seiner Drehachse angeordnet ist, der geringer ist als die Länge eines Fördertaktes der Fördereinrichtung 11. Weiterhin ist die Drehachse der Transportsterne 55 in einer Höhe angeordnet, die der Höhe der oberen Aufnahmesegmente 42 auf den Aufnahmeplatten 38 entspricht. Mittels der zweiten Umsetzeinrichtung 29 werden die oberen Aufnahmesegmente 42 wieder auf die unteren Aufnahmesegmente 41 mit den vollständig befüllten Kapselunterteilen 2 aufgesetzt.

Die in der Fig. 14 dargestellte Schließeinrichtung 30 an der Station L, die sich an die zweite Umsetzeinrichtung 29 anschließt, schiebt die Kapselunterteile 2 mittels auf- und abbewegbarer Stifte 84 in Richtung der Kapseloberteile 3, wobei die Kapseln 1 wieder verschlossen werden. Hierbei liegen die Kapseloberteile 3 an einer Gegenhalterplatte 85 an. Hierdurch wird vermieden, dass beim Gegeneinanderschieben von Kapselunterteilen 2 und Kapseloberteilen 3 die Kapseloberteile 3 aus ihren oberen Aufnahmebohrungen 44 angehoben werden.

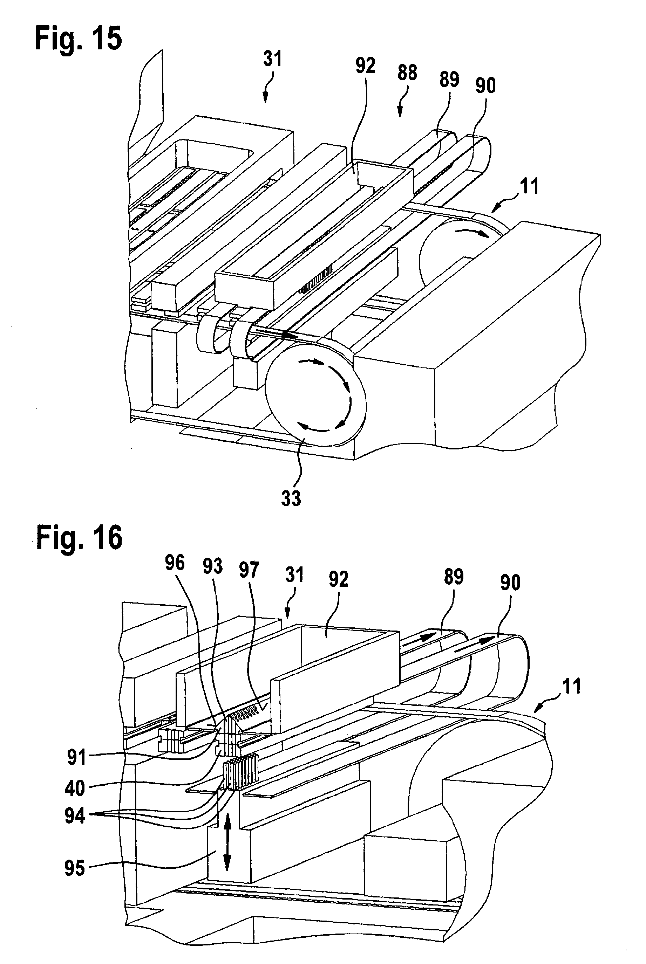

In den Fig. 15 und 16 ist die Ausscheideeinrichtung 31 an der Station M dargestellt. Die Ausscheideeinrichtung 31 umfasst hierbei eine weitere Fördereinrichtung 88 mit zwei, parallel zueinander angeordneten Transportbändern 89, 90. Die Transportbänder 89, 90 dienen hierbei dem Abtransport von Schlecht-Kapseln 1. Die Ausscheideeinrichtung 31 weist ein Ausschiebeprisma 91 auf, das während einer Stillstandsphase der Fördereinrichtung 11 in Ausrichtung mit den Aufnahmeeinheiten 40 angeordnet ist. Ferner ist das Ausschiebeprisma 91 zwischen den beiden Transportbändern 89, 90 angeordnet und von einem Schutzrahmen 92 umgeben, der verhindert, dass Schlecht-Kapseln 1 von den Transportbändern 89, 90 in den unteren Bereich der Vorrichtung 10 herabfallen. Das Ausschiebeprisma 91 weist Durchgangsbohrungen 93 auf, die mit den Aufnahmebohrungen 43, 44 der Aufnahmeeinheiten 40 ausgerichtet sind. Ferner wirken die Durchgangsbohrungen 93 mit Ausschiebestempeln 94 zusammen, die an einem auf- und abbewegbaren Träger 95 befestigt sind und (je nach Ausführung der Vorrichtung 10) einzeln, reihenweise oder komplett bewegbar sind. Die Durchgangsbohrungen 93 des Ausschiebeprismas 91 münden in gegen die Horizontale schräg angeordneten Rutschflächen 96, 97, die unmittelbar an die Transportbändern 89, 90 anschließen. Zum Ausscheiden von Schlecht-Kapseln 1 werden die Ausschiebestempel 94 in einer Stillstandsphase der Fördereinrichtung 11 angehoben und tauchen dabei in die Aufnahmebohrungen 43, 44 der Aufnahmeeinheiten 40 ein. Beim weiteren Anheben der Ausschiebestempel 94 werden die Schlecht-Kapseln 1, ggf. zusammen mit fehlerfreien Kapseln 1, in die Durchgangsbohrungen 93 des Ausschiebeprismas 91 übergeschoben und von dort über die Rutschflächen 96, 97 auf die Transportbändern 89, 90 überführt. Hierbei werden die Transportbänder 89, 90 ggf. nur dann in Betrieb genommen, wenn Schlecht-Kapseln 1 ausgeschieden werden müssen.

Die soweit beschriebene Vorrichtung 10 arbeitet wie folgt: Zunächst werden im Bereich der Sortiereinrichtung 17 mittels der Sortierer 50 geschlossene Kapseln 1 in die übereinander angeordneten Aufnahmesegmente 41, 42 der Aufnahmeeinheiten 40 eingeschoben und deren Kapseloberteile 3 von den Kapselunterteilen 2 getrennt. Anschließend erfolgt mittels der ersten Umsetzeinrichtung 18 das Trennen der oberen Aufnahmesegmente 42 von den unteren Aufnahmesegmenten 41. Sollte es im Bereich der Sortierer 50 hierbei vorgekommen sein, dass Kapseln 1 nicht korrekt in Aufnahmebohrungen 43, 44 eingesetzt wurden, so kann im Bereich der Fehlkapselausscheideeinrichtung 19 die entsprechende Kapsel 1, oder eine Vielzahl von Kapseln 1, ausgeschieden werden. Anschließend werden die Kapselunterteile 2 im Bereich der beiden Fülleinrichtungen 21, 22 vorzugsweise mit unterschiedlichem Füllgut bzw. Produkt 5 befüllt. Im Bereich der Prüfeinrichtung 23 erfolgt anschließend eine Überprüfung der Kapselunterteile 2 auf vorhandenes Füllgut bzw. Produkt 5. Im Bereich der nachfolgenden Fülleinrichtungen 24 bis 27 werden weitere Füllgüter in die Kapselunterteile 2 eindosiert. Anschließend werden die soweit befüllten Kapselunterteile 2 mittels der zweiten Umsetzeinrichtung 29 an der Station K wieder mit den Kapseloberteilen 3 zusammengeführt. Im Bereich der Schließeinrichtung 30 erfolgt anschließend das Aufschieben der Kapselunterteile 2 in die Kapseloberteile 3. Anschließend können an der Ausscheidestation 31 Schlecht-Kapseln 1 ausgeschieden werden. Die im Bereich der Umlenkräder 15 angeordnete Ausschleuseinrichtung 34 (Station N) dient zum nachfolgenden Ausschieben bzw. Ausschleusen von Gut-Kapseln 1 aus den Aufnahmeeinheiten 40 mittels an sich bekannter pneumatisch oder mechanisch wirkender Mittel. Die Aufnahmeeinheiten 40 werden anschließend unterhalb der Stationen A bis M zurückgefördert und gelangen in den Bereich der Reinigungseinrichtung 35 (Station O), wo die Aufnahmebohrungen 43, 44 gereinigt werden, was beispielsweise mittels pneumatischer Einrichtungen erfolgt. Hierbei können ggf. nicht ausgeschiedene Kapseln 1 ebenfalls aus den Aufnahmeeinheiten 40 ausgestoßen werden. Anschließend wiederholt sich der Füll- und Verschließvorgang wie beschrieben.

Die soweit beschriebene Vorrichtung 10 kann in vielfältiger Art und Weise abgewandelt bzw. modifiziert werden, ohne vom Erfindungsgedanken abzuweichen. Dieser besteht insbesondere in dem Vorsehen einer linear ausgebildeten Fördereinrichtung 11 mit einer Vielzahl von Fülleinrichtungen 21, 22 sowie 24 bis 27, die sich in Förderrichtung 36 der Fördereinrichtung 11 hintereinander anschließen.

## Patentansprüche

1. Vorrichtung (10) zum Füllen und Verschließen von mit wenigstens einem Füllgut befüllten Kapseln (1), mit einer insbesondere taktweise angetriebenen Fördereinrichtung (11), mit an der Fördereinrichtung (11) in gleichmäßigen Abständen zueinander angeordneten aus jeweils einem unteren und einem oberen Aufnahmesegment (41, 42) bestehenden Aufnahmeeinheiten (40), die jeweils eine Vielzahl von Kapselunterteilen (2) und Kapseloberteilen (3) aufnehmen, einer Einrichtung (17) zum Zuführen von leeren Kapseln (1) in Aufnahmebohrungen (43, 44) der unteren und oberen Aufnahmesegmente (41, 42), mehreren Fülleinrichtungen (21, 22, 24 bis 27), die jeweils eine bestimmte Menge eines bestimmten Füllguts in die Kapselunterteile (2) eindosieren, einer Umsetzeinrichtung (29) zum Aufsetzen der oberen Aufnahmesegmente (42) auf die mit den befüllten Kapselunterteilen (2) ausgestatteten unteren Aufnahmesegmente (41) und einer Ausschleusseinrichtung (34) für die befüllten Kapseln (1), wobei die Fördereinrichtung (11) mindestens ein endlos ausgebildetes Fördertrum (12, 13), insbesondere einen Fördergurt oder eine Förderkette aufweist, an welchem die Aufnahmeeinheiten (40) mittels insbesondere rechteckförmiger Aufnahmeplatten (38) befestigt sind, **dadurch gekennzeichnet**, mehrere Fülleinrichtungen (21, 22, 24 bis 27) entlang einer geradlinig ausgebildeten Förderstrecke der Kapseln (1) in Förderrichtung (36) nacheinander angeordnet sind.

2. Vorrichtung nach Anspruch 1,
**dadurch gekennzeichnet,**
**dass** die leeren Kapseln (1) als vorverschlossene Kapseln (1) der Einrichtung (17) zugeführt werden, dass die unteren und oberen Aufnahmesegmente (41, 42) zunächst übereinander angeordnet sind und, dass der Einrichtung (17) eine erste Umsetzeinrichtung (18) nachgeordnet ist, die die oberen Aufnahmesegmente (42) von den unteren Aufnahmesegmenten (41) trennt.

3. Vorrichtung nach Anspruch 1 oder 2,
**dadurch gekennzeichnet,**
**dass** die Fülleinrichtungen (21, 22, 24 bis 27) unterschiedliche Füllgüter, insbesondere ein Saatgut sowie weitere Zusatzstoffe wie Dünger, Pestizide o.ä., in die Kapselunterteile (2) eindosieren.

4. Vorrichtung nach einem der Ansprüche 1 bis 3,
**dadurch gekennzeichnet,**
**dass** die Aufnahmeplatten (38) an dem mindestens einen Fördertrum (12, 13) austauschbar befestigt sind.

5. Vorrichtung nach einem der Ansprüche 1 bis 4,
**dadurch gekennzeichnet,**
**dass** die Fördereinrichtung (11) wenigstens zwei Fördertrume (12, 13) aufweist, dass die Aufnahmeplatten (38) die wenigstens zwei Fördertrume (12, 13) überspannen und, dass je Aufnahmeplatte (38) insbesondere mehrere, quer zur Förderrichtung (36) zueinander fluchtend angeordnete Aufnahmeeinheiten (40) an den Aufnahmeplatten (38) angeordnet sind.

6. Vorrichtung nach einem der Ansprüche 1 bis 5,
**dadurch gekennzeichnet,**
**dass** in jeder der Aufnahmeeinheiten (40) mehrere, in Förderrichtung (36) nacheinander angeordnete Reihen (45 bis 48) mit Aufnahmebohrungen (43, 44) für die Kapseln (1) ausgebildet sind und, dass jeder Reihe (45 bis 48) von Aufnahmebohrungen (43, 44) eine separate Sortiereinrichtung (50) zum Zuführen der leeren Kapseln (1) zugeordnet ist, wobei die separaten Sortiereinrichtungen (50) in Förderrichtung (36) jeweils einen Abstand zueinander aufweisen, der der Länge eines Fördertaktes der Fördereinrichtung (11), verringert oder verlängert um den Abstand zweier Reihen (45 bis 48) an Aufnahmebohrungen (43, 44) zueinander entspricht.

7. Vorrichtung nach einem der Ansprüche 2 bis 6,
**dadurch gekennzeichnet,**
**dass** beide Umsetzeinrichtungen (18, 29) zum Trennen bzw. wieder Zusammenführen der Aufnahmesegmente (41, 42) für jede Aufnahmeeinheit (40) zwei, an gegenüberliegenden Seiten der Aufnahmeeinheit (40) angeordnete Transportsterne (55) aufweist, die in einer horizontalen Achse (64) drehbar gelagert sind, wobei die Transportsterne (55) jeweils wenigstens einen Förderarm (56 bis 59) aufweisen, der mit einem oberen Aufnahmesegment (42) zusammenwirkt und dieses aufnimmt, wobei die Länge des Förderarms (56 bis 59) der Länge eines Fördertakts der Fördereinrichtung (11), verringert oder verlängert um wenigstens die Breite der unteren Aufnahmesegmente (41) in Förderrichtung (36) entspricht.

8. Vorrichtung nach Anspruch 6 oder 7,
**dadurch gekennzeichnet,**
**dass** die Reihen (45 bis 48) der Aufnahmebohrungen (43, 44) senkrecht zur Förderrichtung (36) betrachtet zueinander versetzt angeordnet sind, dass wenigstens eine Fülleinrichtung (26) einen Produktspeicherbehälter (65) mit einem unterhalb des Produktspeicherbehälters (65) angeordneten, in und entgegen der Förderrichtung (36) bewegbaren Dosierschieber (68) aufweist, wobei der Dosierschieber (68) Durchgangsbohrungen (67) aufweist, deren Anordnung der Anordnung der Aufnahmebohrungen (43) in den unteren Aufnahmesegmenten (41) entspricht, dass der Dosierschieber (68) mit einer unterhalb des Dosierschiebers (68) angeordneten, ortsfesten Dosierplatte (70) zusammenwirkt, die in Ausrichtung mit den Kapselunterteilen (2) Durchgangsbohrungen (71) aufweist, durch die das in den Durchgangsbohrungen (69) des Dosierschiebers (68) befindliche Füllgut in die Kapselunterteile (2) gelangt und, dass der Verfahrweg des Dosierschiebers (68) dem Abstand zweier Reihen (45 bis 48) entspricht.

9. Vorrichtung nach einem der Ansprüche 1 bis 6,
**dadurch gekennzeichnet,**
**dass** der Ausschleusseinrichtung (34) für die befüllten Gut-Kapseln eine Ausscheideeinrichtung (31) für Schlecht-Kapseln vorgeordnet ist und, dass die Ausscheideeinrichtung (31) Ausschiebestempel (94) aufweist, die zumindest die Schlecht-Kapseln aus den Aufnahmebohrungen (43, 44) der Aufnahmeeinheiten (40) auf eine zusätzliche Fördereinrichtung (88) zumindest mittelbar ausschiebt, die die Schlecht-Kapseln aus dem Bereich der Fördereinrichtung (11) fördert.

10. Vorrichtung nach Anspruch 9,
**dadurch gekennzeichnet,**
**dass** die Ausscheideeinrichtung (31) ein Ausschiebeprisma (91) aufweist, das in einer Stillstandsphase der Fördereinrichtung (11) in Überdeckung mit einer Aufnahmeeinheit (40) und oberhalb der Aufnahmeeinheit (40) angeordnet ist, dass das Ausschiebeprisma (91) Durchgangsbohrungen (93) aufweist, die in Deckung mit den Aufnahmebohrungen (43, 44) der Aufnahmeeinheit (40) angeordnet sind und, dass das Prisma (91) wenigstens eine insbesondere schräg zur Horizontalen angeordnete Rutschfläche (96, 97) aufweist, von der Schlecht-Kapseln auf die weitere Fördereinrichtung (88) gelangen.

11. Vorrichtung nach Anspruch 10,
**dadurch gekennzeichnet,**
**dass** zwei Rutschflächen (96, 97) auf gegenüberliegenden Seiten des Ausschiebeprismas (91) vorgesehen sind, die mit unterschiedlichen Reihen (45 bis 48) von Aufnahmebohrungen (43, 44) zusammenwirken.

12. Vorrichtung nach Anspruch 10 oder 11,
**dadurch gekennzeichnet,**
**dass** jeder der Rutschflächen (96, 97) ein separates Transportband (89, 90) der zusätzlichen Fördereinrichtung (88) zugeordnet ist.

13. Vorrichtung nach einem der Ansprüche 1 bis 12,
**dadurch gekennzeichnet,**
**dass** die Ausschleusseinrichtung (34) für die befüllten Kapseln (1) an einem Umkehrbereich der Fördereinrichtung (11) angeordnet ist.

14. Vorrichtung nach einem der Ansprüche 1 bis 13,
**dadurch gekennzeichnet,**
**dass** die Kapseln (1) als auflösende Kapseln (1) ausgebildet sind.

## Claims

1. Device (10) for filling and closing capsules (1) filled with at least one filling material, having an, in particular, cyclically driven conveying unit (11), having receiving units (40) which are arranged on the conveying unit (11) at regular distances from one another and in each case consist of one lower and one upper receiving segment (41, 42) and which in each case receive a multiplicity of capsule lower parts (2) and capsule upper parts (3), and a unit (17) for infeeding empty capsules (1) into receiving borings (43, 44) of the upper and lower receiving segments (41, 42), a plurality of filling units (21, 22, 24 to 27) which in each case dose a specific amount of a specific filling material into the capsule lower parts (2), a transfer unit (29) for placing the upper receiving segments (42) onto the lower receiving segments (41) which are stocked with the filled capsule lower parts (2), and a discharge unit (34) for the filled capsules (1), wherein the conveying unit (11) has at least one continuously configured conveying strand (12, 13), in particular a conveyor belt or a conveyor chain, on which the receiving units (40) are fastened by means of, in particular, rectangular receiving plates (38),
**characterized**
**in that** a plurality of filling units (21, 22, 24 to 27) are arranged behind one another in the conveying direction (36) along a linearly configured conveying path of the capsules (1).

2. Device according to Claim 1,
**characterized**
**in that** the empty capsules (1) are infed as pre-closed capsules (1) to the unit (17), in that the lower and upper receiving segments (41, 42) are initially arranged on top of one another, and in that a first transfer unit (18), which separates the upper receiving segments (42) from the lower receiving segments (41), is arranged downstream of the unit (17).

3. Device according to Claim 1 or 2,
**characterized**
**in that** the filling units (21, 22, 24 to 27) dose various filling materials, in particular seeds and other additives, such as fertilizers, pesticides and similar, into the capsule lower parts (2).

4. Device according to one of Claims 1 to 3,
**characterized**
**in that** the receiving plates (38) are fastened in an exchangeable manner on the at least one conveying strand (12, 13).

5. Device according to one of Claims 1 to 4,
**characterized**
**in that** the conveying unit (11) has at least two conveying strands (12, 13), in that the receiving plates (38) span the at least two conveying strands (12, 13) and in that for each receiving plate (38), in particular, a plurality of receiving units (40), which are arranged transversely to the conveying direction (36) and aligned to be flush with one another, are arranged on the receiving plates (38).

6. Device according to one of Claims 1 to 5,
**characterized**
**in that** a plurality of rows (45 to 48), which are arranged behind one another in the conveying direction (36) and have receiving borings (43, 44) for the capsules (1), are configured in each of the receiving units (40), and in that each row (45 to 48) of receiving borings (43, 44) is assigned a separate sorting unit (50) for infeeding the empty capsules (1), wherein the separate sorting units (50) in each case, in the conveying direction (36), have a distance from one another which corresponds to the length of a conveying cycle of the conveying unit (11), reduced or increased by the distance of two rows (45 to 48) of receiving borings (43,44) from one another.

7. Device according to one of Claims 2 to 6,
**characterized**
**in that** both transfer units (18, 29), for separating, or reuniting, respectively, the receiving segments (41, 42), have for each receiving unit (40) two transport star wheels (55) which are arranged on opposite sides of the receiving unit (40) and which are rotatably mounted in a horizontal axis (64), wherein the transport star wheels (55) have in each case at least one conveying arm (56 to 59) which interacts with and receives an upper receiving segment (42), wherein the length of the conveying arm (56 to 59) corresponds to the length of a conveying cycle of the conveying unit (11), reduced or increased in the conveying direction (36) by at least the width of the lower receiving segments (41).

8. Device according to Claim 6 or 7,
**characterized**
**in that** the rows (45 to 48) of the receiving borings (43, 44), when viewed perpendicularly to the conveying direction (36), are arranged offset in relation to one another, in that at least one filling unit (26) has a product hopper (65) having a dosing slide (68) which is arranged below the product hopper (65) and is movable in and counter to the conveying direction (36), wherein the dosing slide (68) has through borings (67) which, in their arrangement, correspond to the arrangement of the receiving borings (43) in the lower receiving segments (41), in that the dosing slide (68) interacts with a stationary dosing plate (70) which is arranged below the dosing slide (68) and has through borings (71) which are in alignment with the capsule lower parts (2) and through which the filling material which is situated in the through borings (69) of the dosing slide (68) arrives at the capsule lower parts (2), and in that the displacement path of the dosing slide (68) corresponds to the distance between two rows (45 to 48).

9. Device according to one of Claims 1 to 6,
**characterized**
**in that** a segregation unit (31) for reject capsules is arranged upstream of the discharge unit (34) for the filled accept capsules, and in that the segregation unit (31) has ejecting dies (94) which at least mediately push at least the reject capsules from the receiving borings (43, 44) of the receiving units (40) onto an additional conveying unit (88) which conveys the reject capsules from the region of the conveying unit (11).

10. Device according to Claim 9,
**characterized**
**in that** the segregation unit (31) has an ejecting prism (91) which, in a static phase of the conveying unit (11), is arranged in superposition with a receiving unit (40) and above the receiving unit (40), in that the ejecting prism (91) has through borings (93) which are arranged in superimposition with the receiving borings (43, 44) of the receiving unit (40), and in that the prism (91) has at least one slide surface (96, 97) which is, in particular, arranged at an incline in relation to the horizontal and from which reject capsules arrive at the further conveying unit (88).

11. Device according to Claim 10,
**characterized**
**in that** two slide surfaces (96, 97), which interact with different rows (45 to 48) of receiving borings (43, 44), are provided on opposite sides of the ejecting prism (91).

12. Device according to Claim 10 or 11,
**characterized**
**in that** each of the slide surfaces (96, 97) is assigned a separate transport belt (89, 90) of the additional conveying unit (88).

13. Device according to one of Claims 1 to 12,
**characterized**
**in that** the discharge unit (34) for the filled capsules (1) is arranged at a reversal region of the conveying unit (11).

14. Device according to one of Claims 1 to 13,
**characterized**
**in that** the capsules (1) are configured as dissolving capsules (1).

## Revendications

1. Dispositif (10) pour remplir et fermer des capsules (1) remplies d'au moins une matière de remplissage, comprenant un système de transport (11) entraîné notamment de manière cadencée, avec des unités de réception (40) constituées à chaque fois d'un segment de réception inférieur et d'un segment de réception supérieur (41, 42), disposées les unes par rapport aux autres à intervalles réguliers au niveau du système de transport (11), qui reçoivent à chaque fois une pluralité de parties inférieures de capsules (2) et de parties supérieures de capsules (3), un système (17) pour acheminer des capsules vides (1) dans des alésages de réception (43, 44) des segments de réception inférieurs et supérieurs (41, 42), plusieurs systèmes de remplissage (21, 22, 24 à 27), qui dosent à chaque fois une quantité déterminée d'une matière de remplissage déterminée dans les parties inférieures de capsules (2), un système de transfert (29) pour poser les segments de réception supérieurs (42) sur les segments de réception inférieurs (41) munis des parties inférieures de capsules remplies (2) et un système d'évacuation (34) pour les capsules remplies (1), le système de transport (11) présentant au moins un tronçon de transport sans fin (12, 13), en particulier une courroie de transport ou une chaîne de transport, au niveau duquel les unités de réception (40) sont fixées au moyen de plaques de réception (38) notamment rectangulaires, **caractérisé en ce que** plusieurs systèmes de remplissage (21, 22, 24 à 27) sont disposés les uns derrière les autres dans la direction de transport le long d'une section de transport des capsules (1) réalisée sous forme rectiligne.

2. Dispositif selon la revendication 1,
**caractérisé en ce que**
les capsules vides (1) sont acheminées au système (17) sous forme de capsules (1) préfermées, **en ce que** les segments de réception inférieurs et supérieurs (41, 42) sont d'abord disposés les uns au-dessus des autres et **en ce qu'**un premier système de transfert (18) est disposé en aval du système (17), lequel premier système de transfert sépare les segments de réception supérieurs (42) des segments de réception inférieurs (41).

3. Dispositif selon la revendication 1 ou 2,
**caractérisé en ce que**
les systèmes de remplissage (21, 22, 24 à 27) dosent différentes matières de remplissage, notamment des semences ainsi que d'autres additifs tels que des engrais, des pesticides ou autres, dans les parties inférieures de capsules (2).

4. Dispositif selon l'une quelconque des revendications 1 à 3,
**caractérisé en ce que**
les plaques de réception (38) sont fixées de manière remplaçable sur l'au moins un tronçon de transport (12, 13).

5. Dispositif selon l'une quelconque des revendications 1 à 4,
**caractérisé en ce que**
le dispositif de transport (11) présente au moins deux tronçons de transport (12, 13), **en ce que** les plaques de réception (38) s'étendent au-dessus des au moins deux tronçons de transport (12, 13) et **en ce que** pour chaque plaque de réception (38), en particulier plusieurs unités de réception (40) disposées de manière alignées les unes avec les autres transversalement à la direction de transport (36) sont disposées sur les plaques de réception (38).

6. Dispositif selon l'une quelconque des revendications 1 à 5,
**caractérisé en ce que**
dans chacune des unités de réception (40) sont réalisées plusieurs rangées (45 à 48) disposées les unes derrière les autres dans la direction de transport (36), avec des alésages de réception (43, 44) pour les capsules (1), et **en ce qu'**à chaque rangée (45 à 48) d'alésages de réception (43, 44) est associé un système de tri séparé (50) pour acheminer les capsules vides (1), les systèmes de tri séparés (50) présentant à chaque fois un espacement les uns par rapport aux autres dans la direction de transport (36), lequel espacement correspond à la longueur d'un cycle de transport du système de transport (11), réduit ou prolongé de la distance mutuelle entre deux rangées (45 à 48) d'alésages de réception (43, 44).

7. Dispositif selon l'une quelconque des revendications 2 à 6,
**caractérisé en ce que**
les deux systèmes de transfert (18, 29) pour séparer ou réunir à nouveau les segments de réception (41, 42) pour chaque unité de réception (40) présentent deux étoiles de transport (55) disposées au niveau de côtés opposés de l'unité de réception (40), lesquelles étoiles de transport sont montées à rotation dans un axe horizontal (64), les étoiles de transport (55) présentant à chaque fois au moins un bras de transport (56 à 59) qui coopère avec un segment de réception supérieur (42) et le reçoit, la longueur du bras de transport (56 à 59) correspondant à la longueur d'un cycle de transport du système de transport (11), réduit ou prolongé d'au moins la largeur des segments de réception inférieurs (41) dans la direction de transport (36).

8. Dispositif selon la revendication 6 ou 7,
**caractérisé en ce que**
les rangées (45 à 48) des alésages de réception (43, 44), considéré perpendiculairement à la direction de transport (36), sont disposées de manière décalées les unes des autres, **en ce qu'**au moins un système de remplissage (26) présente un récipient de stockage de produit (65) avec un tiroir de dosage (68) disposé sous le récipient de stockage de produit (65) et déplaçable dans la direction de transport (36) et dans la direction opposée, le tiroir de dosage (68) présentant des alésages traversants (67) dont l'agencement correspond à l'agencement des alésages de réception (43) dans les segments de réception inférieurs (41), **en ce que** le tiroir de dosage (68) coopère avec une plaque de dosage fixe (70) disposée en dessous du tiroir de dosage (68), qui présente des alésages traversants (71) alignés avec les parties inférieures de capsules (2), à travers lesquels alésages la matière de remplissage se trouvant dans les alésages traversants (69) du tiroir de dosage (68) parvient dans les parties inférieures de capsules (2) et **en ce que** la course de déplacement du tiroir de dosage (68) correspond à la distance entre deux rangées (45 à 48).

9. Dispositif selon l'une quelconque des revendications 1 à 6,
**caractérisé en ce que**
le système d'évacuation (34) pour les capsules de matière remplies est précédé d'un système de séparation (31) pour les capsules défectueuses, et **en ce que** le système de séparation (31) présente des poinçons d'éjection (94) qui évacuent au moins indirectement au moins les capsules défectueuses hors des alésages de réception (43, 44) des unités de réception (40) vers un système de transport supplémentaire (88) qui transporte les capsules défectueuses hors de la région du système de transport (11) .

10. Dispositif selon la revendication 9,
**caractérisé en ce que**
le système de séparation (31) présente un prisme d'évacuation (91) qui, dans une phase d'arrêt du système de transport (11), est disposé de manière coïncidente avec une unité de réception (40) et au-dessus de l'unité de réception (40), **en ce que** le prisme d'évacuation (91) présente des alésages traversants (93) qui sont disposés en coïncidence avec les alésages de réception (43, 44) de l'unité de réception (40) et **en ce que** le prisme (91) présente au moins une surface de glissement (96, 97) disposée notamment obliquement par rapport à l'horizontale, depuis laquelle les capsules défectueuses arrivent au système de transport supplémentaire (88).

11. Dispositif selon la revendication 10,
**caractérisé en ce que**
deux surfaces de glissement (96, 97) sont prévues sur des côtés opposés du prisme d'évacuation (91), lesquelles coopèrent avec des rangées différentes (45 à 48) d'alésages de réception (43, 44).

12. Dispositif selon la revendication 10 ou 11, **caractérisé en ce que**
chacune des surfaces de glissement (96, 97) est associée à une bande transporteuse séparée (89, 90) du système de transport supplémentaire (88).

13. Dispositif selon l'une quelconque des revendications 1 à 12,
**caractérisé en ce que**
le système d'évacuation (34) pour les capsules remplies (1) est disposé au niveau d'une région d'inversion du système de transport (11).

14. Dispositif selon l'une quelconque des revendications 1 à 13,
**caractérisé en ce que**
les capsules (1) sont réalisées sous forme de capsules solubles (1).
